# EUROPÄISCHE PATENTSCHRIFT

(11) **EP 0 336 009 B1**
(45) Veröffentlichungstag und Bekanntmachung des Hinweises auf die Patenterteilung: **29.12.1993**
(21) Anmeldenummer: 88117447.8
(22) Anmeldetag: 20.10.1988
(51) Int. Cl.: A61B 17/36

(54) **Vorrichtung an einem Lichtleiter**
Single light conductor device
Dispositif à un conduit de lumière

(30) Priorität: 07.03.1988 DE 3807437
(43) Veröffentlichungstag der Anmeldung: 11.10.1989
(73) Patentinhaber: Deutsche Aerospace AG, 81663 München (DE)
(72) Erfinder: Frank, Frank, Dr., D-8017 Ebersberg (DE); Hengst, Thomas, D-8013 Haar (DE); Hahn, Andreas, D-8029 Sauerlach (DE)

(56) Entgegenhaltungen:
- EP-A- 0 069 389
- EP-A- 0 194 841
- GB-A- 2 177 518
- US-A- 4 537 193

## Beschreibung

Die Erfindung betrifft eine Vorrichtung zur Längsverschiebung der Lichtleitermäntel (4, 5) eines Lichtleiters (1) für die Verbindung eines medizinischen Lasergeräts mit einem Endoskop, wobei der Lichtleiter (1) an dem Ende, an welches das Endoskop anschließbar ist, eine Vorrichtung (2) zur Längsverschiebung des Lichtleiters (1) aufweist.

Ein derartiger Lichtleiter ist in der DE-PS 29 45 080 beschrieben. Der Lichtleiter weist hierbei am Anschlußstück zum Endoskop eine Vorrichtung zur Längsverschiebung des Lichtleiters auf, die es ermöglicht, das distale Ende des Lichtleiters um einige Millimeter zwangsläufig reversibel aus dem Endoskop austreten zu lassen, um die Lichtleiterspitze während einer Behandlung reinigen zu können. Eine Längenänderung des austretenden Lichtleiters um eine Strecke von bis zu einem Meter ist jedoch mit dieser Vorrichtung nicht möglich.

Aus der DE-OS 21 45 921 ist ein Lichtleiter bekannt geworden, bei dem die Lichtleitfaser in einem Tubus angeordnet ist, der bezüglich eines weiteren Tubus teleskopartig reversibel verschiebbar gelagert ist. Eine Längenverstellung des Lichtleiters ist jedoch nicht vorgesehen.

Der Erfindung liegt deshalb die Aufgabe zugrunde, eine Lichtleiterverbindung zwischen einem medizinischen Lasergerät und einem Endoskop so zu gestalten, daß der aus dem Anschlußstück zum Endoskop austretende Lichtleiter auf beliebige Längen bis zu einem Meter einstellbar ist, so daß der Lichtleiter an verschiedene Endoskope angepaßt werden kann, und daß die bekannte Vorrichtung zur Längsverschiebung des Lichtleiters weiterhin ihre Funktion erfüllen kann.

Diese Aufgabe wird erfindungsgemäß durch die im kennzeichnenden Teil des Patentanspruches wiedergegebenen Merkmale gelöst.

Der besondere Vorteil der Vorrichtung ist es, daß für jedes auf dem Markt befindliche Endoskop mit passendem Anschluß jede beliebige Länge des aus dem Anschlußstück austretenden Lichtleiters einstellbar ist, so daß sich dadurch die Lagerhaltung für Lichtleiter drastisch reduzieren läßt. Außerdem kann ein Lichtleiter über längere Zeit durch einfaches Abschneiden seiner distalen Spitze immer wieder benutzt werden, während bisher im Falle einer Beschädigung jeweils der gesamte Lichtleiter ausgetauscht werden mußte. Weiterhin ergibt sich durch den steifen Lichtleitermantel ein guter Knickschutz und eine Zugentlastung für den Lichtleiter.

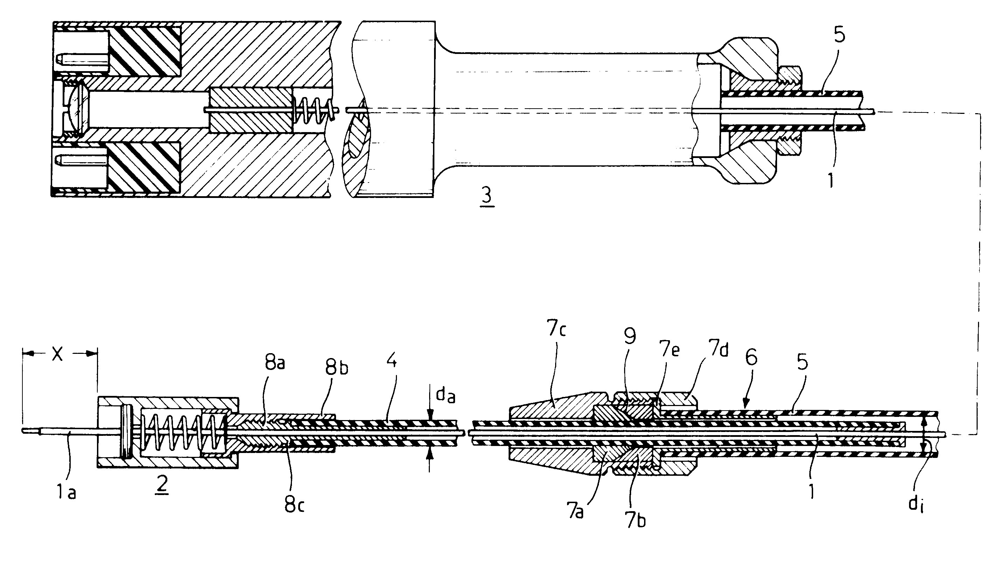

Ein Ausführungsbeispiel der Erfindung ist nachfolgend anhand der Zeichnung beschrieben. Die einzige Figur der Zeichnung zeigt schematisch vereinfacht den Aufbau des erfindungsgemäß in der Länge einstellbaren Lichtleiters.

Im Anschlußstück 3 zum nicht dargestellten medizinischen Lasergerät sind der Lichtleiter 1 und der Lichtleitermantel 5 befestigt. In den größeren Lichtleitermantel 5, der den Innendurchmesser dᵢ aufweist, ist an der Übergangsstelle 6 der kleinere Lichtleitermantel 4, der den Außendurchmesser dₐ < dᵢ aufweist, eingeschoben. Beide Lichtleitermäntel bestehen aus biegesteifem Material, beispielsweise aus einer mit Kunststoff überzogenen Metallspirale. Innerhalb der Lichtleitermäntel ist der Lichtleiter 1 lose gelagert.

Die Verklemmung der beiden Lichtleitermäntel 4, 5 gegeneinander erfolgt mittels der ersten lösbaren Klemmverbindung 7a, ... 7e. Der größere Lichtleitermantel 5 ist auf eine Kunststoffhülse 7e geschoben und mit dieser verklebt. Die Klemmung wird durch zwei Klemmhülsen 7a, 7b aus Kunststoff vollzogen, die mittels einer konischen Trennfläche 9 aneinander anliegen. Die im Konusbereich innenliegende Hülse 7a kann geschlitzt ausgeführt sein. Die Klemmung tritt ein, wenn die beiden Metallhülsen 7c, 7d, die die Kunststoffhülse 7e und die Klemmhülsen 7a, 7b umfassen, gegeneinander verschraubt werden, so daß die beiden Klemmhülsen 7a, 7b in axialer Richtung aufeinander geschoben werden.

Die weitere lösbare Klemmverbindung an der Vorrichtung zur Längsverschiebung des Lichtleiters 2, die aus den Teilen 8a, ..., 8c besteht, ist prinzipiell gleichartig wie die erste lösbare Klemmverbindung 7a, ..., 7e aufgebaut und verbindet den Lichtleiter 1 mit dem kleineren Lichtleitermantel 4.

Mit Hilfe der vorbeschriebenen Vorrichtung kann nun leicht die Länge X des aus der Vorrichtung zur Längsverschiebung 2 austretenden Lichtleiters 1a innerhalb der durch die Länge des kleineren und des größeren Lichtleitermantels 4, 5 vorgegebenen Bereichs eingestellt werden. Hierzu wird zunächt die Klemmung an der Vorrichtung zur Längsverschiebung 2 und dann die Klemmung an der übergangsstelle 6 gelöst. Nach der erfolgten Einstellung der Länge X erfolgt die Klemmung in der umgekehrten Reihenfolge.

## Patentansprüche

1. Vorrichtung zur Längsverschiebung der Lichtleitermäntel (4, 5) eines Lichtleiters (1) für die Verbindung eines medizinischen Lasergeräts mit einem Endoskop, wobei der Lichtleiter (1) an dem Ende, an welches das Endoskop anschließbar ist, eine Vorrichtung (2) zur Längsverschiebung des Lichtleiters (1) aufweist, **gekennzeichnet** durch folgende Merkmale:
a) der Lichtleiter (1) ist zwischen der Vorrichtung zur Längsverschiebung (2) und dem Einkoppelstück (3) zum Lasergerät in einem ersten und einem zweiten steifen Lichtleitermantel (4, 5) angeordnet, wobei der Außendurchmesser dₐ des ersten Lichtleitermantels (4) kleiner als der Innendurchmesser dᵢ des zweiten Lichtleitermantels (5) ist so daß an einer Übergangsstelle (6) zwischen dem ersten und dem zweiten Lichtleitermantel der erste Lichtleitermantel (4) koaxial in den zweiten Lichtleitermantel (5) schiebbar ist;
b) der erste und der zweite Lichtleitermantel (4, 5) sind an der Übergangsstelle (6) mittels einer ersten lösbaren Klemmeinrichtung (7a ,..., 7e) verbunden;
c) an der Vorrichtung zur Längsverschiebung (2) ist der Lichtleiter (1) mittels einer weiteren lösbaren Klemmeinrichtung (8a, ..., 8c) mit dem den Außendurchmesser dₐ aufweisenden Lichtleitermantel (4) verbunden.

## Claims

1. Device for longitudinal displacement of the fibre-optic casings (4, 5) of an optical fibre (1) for the connection of a medical laser unit to an endoscope, in which respect the optical fibre (1) comprises at the end to which the endoscope is coupled a device (2) for longitudinal displacement of the optical fibre (1), **characterised by** the following features:
a) the optical fibre (1) is arranged between the device for longitudinal displacement (2) and the coupling element (3) to the laser unit in a first and a second stiff optical fibre sleeve (4, 5), in which respect the outside diameter dₐ of the first optical fibre casing (4) is smaller than the inside diameter dᵢ of the second optical fibre casing (5), so that the first optical fibre casing (4) is coaxially slidable into the second optical fibre casing (5) at a transitional place (6) between the first and the second optical fibre casing;
b) the first and the second optical fibre casing (4, 5) are connected at the transitional place (6) by means of a first releasable clamping device (7a, ..., 7e);
c) the optical fibre (1) is connected on the device for longitudinal displacement (2) by means of a further releasable clamping device (8a, ..., 8c) to the optical fibre casing (4) of outside diameter dₐ.

## Revendications

1. Dispositif pour déplacer dans la direction longitudinale la gaine (4, 5) d'une fibre optique (1) pour la connexion d'un appareil laser médical avec un endoscope, la fibre optique (1) présentant à son extrémité à laquelle l'endoscope peut être connecté, un dispositif (2) pour déplacer la fibre optique (1) dans la direction longitudinale, caractérisé par les caractéristiques suivantes:
a) entre le dispositif (2) destiné à assurer le déplacement dans la direction longitudinale et l'élément de couplage (3) avec l'appareil laser, la fibre optique (1) est disposée dans une première et une deuxième gaine rigide (4, 5), le diamètre extérieur dₐ de la première gaine (4) de fibre optique étant inférieur au diamètre intérieur dᵢ de la deuxième gaine (5) de fibre optique de telle sorte qu'en un point de transition (6) entre la première et la deuxième gaine de fibre optique, la première gaine (4) de fibre optique puisse se déplacer coaxialement dans la deuxième gaine (5) de fibre optique;
b) les première et la deuxième gaines (4, 5) de fibre optique sont liées au niveau du point de transition (6) au moyen d'un premier dispositif de serrage (7a,..., 7e) démontable;
c) au niveau du dispositif de déplacement dans la direction longitudinale (2) la fibre optique (1) est liée au moyen d'un autre dispositif de serrage (8a,..., 8c) à la gaine de fibre optique (4) de diamètre extérieur dₐ.
